# EUROPÄISCHE PATENTSCHRIFT

(11) **EP 0 692 959 B1**
(45) Veröffentlichungstag und Bekanntmachung des Hinweises auf die Patenterteilung: **04.10.2000**
(21) Anmeldenummer: 94912509.0
(22) Anmeldetag: 23.03.1994
(51) Int. Cl.: A61K 7/48, A61K 33/06, A61K 33/14

(54) **VERWENDUNG VON WASSERLÖSLICHEN MAGNESIUMSALZEN UND ÄUSSERLICH ANZUWENDENDE ZUBEREITUNGEN**
USE OF WATER-SOLUBLE MAGNESIUM SALTS AND PREPARATIONS FOR EXTERNAL APPLICATION
UTILISATION DE SELS DE MAGNESIUM SOLUBLES DANS L'EAU ET PREPARATIONS TOPIQUES

(30) Priorität: 02.04.1993 DE 4310816
(43) Veröffentlichungstag der Anmeldung: 24.01.1996
(73) Patentinhaber: Wogerpharm GmbH, D-50354 Hürth (DE)
(72) Erfinder: DIEZEL, Wolfgang, D-10117 Berlin (DE)
(74) Vertreter: Werner, Hans-Karsten, Dr.Dipl.-Chem.
(86) Internationale Anmeldenummer: EP9400910
(87) Internationale Veröffentlichungsnummer: WO9422421

(56) Entgegenhaltungen:
- EP-A- 0 399 909
- EP-A- 0 565 007
- DD-A- 297 062
- DE-A- 3 800 971
- DE-C- 3 327 840
- DE-C- 4 315 866
- FR-A- 2 122 613
- FR-A- 2 697 750
- FR-M- 131
- S.T.N., File Supplier, KARLSRUHE, DE, File Chemical Abstracts, vol 113 n 29127 , see the abstract, & JP-A-02011518, publ 16-1-90, TAKIZAWA
- S.T.N., File Supplier, KARLSRUHE, DE, File Chemical Abstracts, vol 95, n 192217, see the abstract, & RO-A-69186, publ 15-7-80, INTREPRINDERA DE PRODUCE COSMETICE MIRAJ

## Beschreibung

Gegenstand der vorliegenden Erfindung sind die Verwendung von wasserlöslichen Magnesiumsalzen in äußerlich anzuwendenden Zubereitungen sowie die Verwendung von wasserlöslichen Magnesiumsalzen zur Herstellung von äußerlichen Zubereitungen sowie neue äußerliche Zubereitungen enthaltend wasserlösliche Magnesiumsalze in Kombination mit Vitamin E und/oder Vitamin E Estern.

Die FR-A-2,122,613 beschreibt pharmazeutische Zubereitungen, welche eine Reihe von Wirkstoffen enthalten, die zusätzlich als Wirkstoffe Kampfer, Natriumchlorid, Calciumchlorid, Magnesiumchlorid oder Magnesiumsulfat und Allantoin oder Komplexe von Aluminium und Allantoin enthalten sollen.

Diese Zubereitungen sollen eingesetzt werden zur Behandlung von verschiedensten Erkrankungen einschließlich zur Behandlung von Sonnenbrand. Es handelt sich somit um Produkte, die bereits eingetretene Schäden behandeln sollen.

Die DE-A-38 00 971 beschreibt eine Salbe mit einem Vitamin/Mineral-Gemisch, welches der Reaktivierung zerstörter Zellen, insbesondere bei Brandschäden oder nach operativen Eingriffen dienen soll. Es handelt sich somit auch um ein Mittel zur Behandlung bereits eingetretener Schäden. Diese Zubereitung enthält neben Magnesium erhebliche Mengen an Calcium, welches erfindungsgemäß kontraproduktiv ist.

Die EP-A-0 399 909 beschreibt eine kosmetische Zubereitung, die Betain, ATP, ein Magnesiumsalz, ein Kaliumsalz und einen kosmetischen Excipienten enthält. Gemäß Anspruch 2 beträgt die Menge an Magnesium nur 10 ppb bis 0.3%. Dies liegt deutlich unter der Menge von 0,5 bis 30 Gew.-% wasserlöslicher Magnesiumsalze.

Die DE-A-33 27 840 beschreibt ein Hautpflegemittel enthaltend Mineralsalze, die Natrium-, Kalium-, Magnesium-, Calcium-, Zink- und Kupferionen liefern. Die Beispiele zeigen, daß das Magnesium meist als Magnesiumsulfat eingesetzt wird und stets erhebliche Mengen an Calciumchlorid vorhanden sind. Calciumchlorid wirkt kontraproduktiv. Die Sonnenschutzcreme gemäß Beispiel 5 enthält einen starken UV-Filter, nämlich 2-Ethoxyethyl-p-methoxycinnamat.

Die DD-A-297,062 geht auf den Erfinder der vorliegenden Erfindung zurück. Es findet sich hierin keinerlei Hinweis auf eine neue Verwendung der Magnesiumsalze. Erwähnt ist lediglich, daß die Wirkung der Magnesiumionen in Kombination mit UV-Licht verstärkt wirksam sind (Balneo-Photo-Therapie).

Die EP-A-0 565 007 (Stand der Technik gemäß AM 54(3) EPÜ) beschreibt ein Badesalz, welches als wirksame Komponente Ascorbinsäure-tocopherolphosphatdiester enthält, wobei die freien Radikale sowohl durch die Ascorbinsäure als auch durch das Tocopherol abgefangen werden sollen. Als weitere wesentliche Komponente enthält das Badesalz Natriumsulfat oder Natriumchlorid. Sofern im Beispiel 3 auch Magnesiumchlorid genannt ist, liegt es in deutlich geringeren Mengen als Natriumchlorid vor. Weiterhin enthält dieses Badesalz Calciumchlorid, von dem bekannt ist, daß es die Wirkung des Magnesiums blockiert.

Die EP-A-0 620 726 (entsprechend FR-A-2697750 und WO-A-94/10974) (Stand der Technik gemäß AM 54(3) EPÜ) beschreibt kosmetische Zubereitungen, die gemäß Anspruch 2 vor allem Aminosäuren, Lecithin, Gelee Royal und Vitamin C enthalten neben 0,02% Spurenelementen. Sofern Vitamin E als weitere Komponente aufgezählt ist, liegt es in Mengen von weniger als 0,25% vor. In diesen geringen Konzentration entwickelt aber Vitamin E keine Hautschutzwirkung mehr.

Aus der EP-A 0 439 640 ist bekannt, daß entzündliche Hauterkrankung und allergisch entzündliche Reaktionen der Haut mit äußerlich anzuwendenden Zubereitungen enthaltend 0,5 bis 30 Gew.-% wässerlöslicher Magnesiumsalze behandelt werden können. Wesentliches Merkmal dieser äußerlichen Zubereitungen ist, daß sie keine oder praktisch keine Calciumionen enthalten, damit das Calcium durch Magnesium verdrängt werden kann und hierdurch die Entzündungsenzyme gehemmt werden. Eine derartige Zubereitung ist von der Anmelderin inzwischen unter der Bezeichnung Wogederm^{R} in den Handel gebracht worden. Sie enthält 25 Gew.-% Magnesiumchlorid, übliche Salbengrundlagen und keine zugesetzten Calciumsalze.

Es wurde jetzt festgestellt, daß derartige Zubereitungen mit mindestens 0,5 Gew.-%, vorzugsweise 5 Gew.-%, wasserlöslichen Magnesiumsalzen obendrein in der Lage sind, UV- Licht-induzierte Schäden der Haut abzuschwächen und dadurch insbesondere die vorzeitige Alterung und die Entstehung von Hautkrebs durch UV-Licht abzuschwächen oder auch ganz zu verhindern. Weiterhin wurde festgestellt, daß die aus der DE-A-35 07 791 bekannte Lichtschutzwirkung von Vitamin E und/oder Vitamin E Estern durch die gleichzeitige Anwesenheit wasserlöslicher Magnesiumsalze deutlich verstärkt wird, so daß es sich anbietet Kombinationspräparate zur Verfügung zu stellen, welche
a) 0,5 bis 30 Gew.-% wasserlösliche Magnesiumsalze und b) 0,4 bis 25 Gew.-% Vitamin E und/oder Vitamin E Ester enthalten.

Gegenstand der vorliegenden Erfindung ist somit die Verwendung von 0,5 bis 30 Gew.-% wasserlöslichen Magnesiumsalzen zur Herstellung von äußerlichen vorbeugenden Zubereitungen gegen UV-Licht-induzierte Schäden der Haut, insbesondere die vorzeitige Alterung und die Entstehung von Hautkrebs sowie Kombinationspräparate gemäß Anspruch 4.

Da die Magnesiumsalze in den äußerlich anzuwendenden Zubereitungen wasserlöslich sein sollen, kommt insbesondere Magnesiumchlorid in Frage. Bereits Magnesiumsulfat ist weniger gut geeignet. Andere wasserlösliche Magnesiumsalze insbesondere organischer Säuren müssen außer der Wasserlöslichkeit auch gut verträglich sein und dürfen vor allem keine allergischen Reaktion oder sonstige Reizungen der Haut auslösen.

Als äußerlich anzuwendende Zubereitungen kommen vor allem in Frage Salben, Cremes, Lotionen, Wässer und Kopfwaschmittel. Es handelt sich somit zunächst um kosmetische Zubereitungen, es sind aber auch Zubereitungen möglich, die nur als Arzneimittel vertrieben werden können. Dies hängt insbesondere davon ab, ob die Hilfs- und Grundstoffe der Zubereitung für Kosmetika zugelassen sind oder nur für Arzneimittel und ob die Zubereitungen auch für andere Zwecke angeboten werden, die eine Heilaussage beinhalten und daher als Arzneimittel deklariert werden.

Die erfindungsgemäß verwendeten Zubereitungen können je nach Verwendungszweck als Wasser in Öl oder Öl in Wasser Emulsionen hergestellt werden, die entweder rasch in die Haut eindringen oder aber längere Zeit als fettiger Film zumindest teilweise auf der Hautoberfläche verbleiben. Aus Lotionen, Wässer und Kopfwaschmitteln dringen die wasserlöslichen Magnesiumsalze im allgemeinen rasch in die Haut ein, so daß sie dort ihre Lichtschutzwirkung entwickeln können. Die Konzentration an Magnesiumsalzen, insbesondere Magnesiumchlorid, kann je nach Anwendungsart zwischen 0,5 und 30 Gew.-% gewählt werden. Vorzugsweise werden Konzentrationen zwischen 5 und 30 Gew.-% gewählt.

Der Gehalt an Vitamin E und/oder Vitamin E Estern kann ebenfalls in weiten Grenzen zwischen 0,4 bis 25 Gew.-% gewählt werden. Vorzugsweise enthalten die Zubereitungen 5 bis 20 Gew.-% Vitamin E und/oder Vitamin E Ester. Bevorzugt werden Zubereitungen, aus denen das Vitamin E und/oder seine Ester gut in die Haut eindringen können gemäß DE B 35 07 791 sowie EP-B 85 102 222.8.

Die erfindungsgemäßen neuen äußerlichen Zubereitungen enthaltend die Kombination aus wasserlöslichem Magnesiumsalz und Vitamin E und/oder Vitamin E Estern, verwenden somit vor allem Salbengrundlagen, die geeignet sind Vitamin E und/oder seinen Estern das Eindringen in die Haut zu ermöglichen. Besonders bevorzugt sind daher Zubereitungen auf Basis von Ölsäureoleylester, die gegebenenfalls noch Emulgatoren und/oder durchblutungsfördende und gefäßerweiternde Substanzen enthalten. In diesen Zubereitungen läßt sich auch Magnesiumchlorid in ausreichenden Mengen einarbeiten, so daß der Doppeleffekt eintreten kann.

Die UV-Licht-induzierten Schäden der Haut, insbesonders die vorzeitige Alterung und die Entstehung von Hautkrebs sind bekannt. Sie werden zurückgeführt auf die Auslösung radikalischer Reaktionen bzw. die UV-Licht-induzierte Lipidperoxydation (R. Hochschild: Exp. Geront. 6 (1971) 153; H. Meffert et al.: Experientia 32 (1976) 1397; T. Günther: Mag.-Bull. 13 (1991) 78 sowie B.D. Goldstein u. G. Witz: Free Rad. Res. Commun. 11 (1990) 3; E.A. Emmelt: CRC Crit. Rev. Toxicol. 2 (1973) 211; F. Urbach: J. Invest. Dermatol. 32 (1959) 373).

Im Verlauf der Lipidperoxidation entstehen Fettsäurehydroperoxiradikale und Lipoperoxide, welche in erheblichem Maße zerfallen in Malondialdehyd (MDA). Malondialdehyd bildet mit Aminogruppen des Gewebes, insbesondere den Aminen, Proteinen und Nukleinsäuren, Schiffsche Basen. Diese durch Malondialdehyd entstandenen Schiffschen Basen können offensichtlich durch zelleigene Enzyme nicht mehr abgebaut werden. Malondialdehyd führt somit zu einer zunehmenden Vernetzung des Kollagens und somit zu einer vorzeitigen Hautalterung. Die durch Malondialdehyd entstehenden Schiffschen Basen von Nukleinsäuren (DNS) sind offensichtlich in erheblichem Maße mit verantwortlich für die Entstehung der UV-B-Licht-induzierten Hautschäden, die von der Zellschädigung bis zum Zelltod oder bis zur Zellentartung führen können.

Es wurde jetzt festgestellt, daß Magnesiumionen in ausreichender Konzentration alleine und in verstärktem Maße in Gegenwart von Vitamin E und/oder Vitamin E Estern die UV-Lichtinduzierte Lipidperoxydation hemmen. Weiterhin wurde nachgewiesen, daß die auf UV-Licht-induzierte DNS-Schädigung zurückgeführte Bildung von sogenannten "Sunborn-cells" bei erfindungsgemäßer zur Verfügungstellung von Magnesiumionen deutlich zurückgeht.

Die Wirkung von Magnesiumionen und Magnesiumionen zusammen mit Vitamin E gehen aus der nachfolgenden Untersuchungsergebnissen hervor:

### Beispiel 1

Operativ entfernte menschliche Haut (2 cm x 1 cm) wurde mit 1,2 J/cm² UV-B-Licht bestrahlt. Jeweils eine Stunde vor der Bestrahlung wurden die in der nachfolgenden Tabelle 1 angegebenen Substanzen aufgetragen. Die aufgetragene Menge betrug jeweils 200 µl auf einem Hautareal von 2 cm x 1 cm. Nach der Bestrahlung wurde die Epidermis mechanisch separiert (E.J. van Scott: J. Invest. Dermatol. 18 (1952) 377). Die gewonnene Epidermis (300 mg) wurde zusammen mit 150 mmol/l KCl homogenisiert und weiter aufgearbeitet (Methodik : R.S. Britton et al.: Hepatology 11 (1990) 93; T. Günther et al.: Mag.-Bull 14 (1992) 57). Es wurde der Gehalt an Malondialdehyd in der Epidermis quantitativ bestimmt. Wie der nachfolgenden Tabelle 1 entnommen werden kann, sind Magnesiumionen befähigt, die UV-Licht-induzierte Malondialdehyd-Bildung zu minimieren. Die kombinierte Behandlung der Haut mit Magnesiumionen + Vitamin E verstärkt diesen Effekt.

**Tabelle 1**

| | n⁴⁾ | Malondialdehyd (µmol/kg) | <p⁵⁾ |
|---|---|---|---|
| Kontrolle (keine UV-B-Bestrahlung | 10 | 2,075 ± 0,15 | |
| UV-B)¹⁾ | 8 | 16,030 ± 2,42 | 0,001 |
| UV-B + Vitamin E²⁾ | 8 | 6,800 ± 1,63 | 0,01 |
| UV-B + MgCl₂ (100 mM)³⁾ | 4 | 13,080 ± 3,51 | 0,001 |
| UV-B + MgCl₂ (500 mM) | 4 | 6,890 ± 1,42 | 0,01 |
| UV-B + MgCl₂ (1000 mM) | 4 | 7,961 ± 1,24 | 0,01 |
| UV-B + Vitamin E + MgCl₂ (500 mM) | 4 | 2,240 ± 0,20 | N.S. |
| UV-B + Vitamin E + MgCl₂ (1000 mM) | 4 | 2,800 ± 0,38 | N.S. |

| | | | |
|---|---|---|---|
| 1) UV-B-Licht: 1,2 J/cm² | | | |
| 2) Vitamin E (Alpha-Tocopherol: 0,5 %) | | | |
| 3) MgCl₂ x 6 H₂O (500 mM = 1 %) | | | |
| 4) Anzahl der untersuchten Hautproben | | | |
| 5) Wilcoxon-Test: statistische Differenz zur Kontrolle N.S.: keine signifikante Differenz | | | |

### Beispiel 2

Verminderte Entstehung UV-Licht-induzierter "sunborn-cells" in menschlicher Epidermis als Folge der Vorbehandlung der Haut mit einer Magnesiumionen enthaltenden Creme (Wogederm^{R} Magnesium-Creme) (Tabelle 2)

Als Folge der äußerlichen Behandlung mit Magnesiumionen entstehen während einer UV-Lichttherapie weniger "suborn-cells" (Tabelle 2). "Sunborn-cells" entstehen durch eine UV-Lichtinduzierte DNS-Schädigung; primär auch durch die Schädigung der DNS als Folge der UV-Licht-indzierten Lipidperoxidation (K. Danno et al.: Photochem. Photobiol. 45 (1987) 683). Derartige UV-Licht-geschädigte Zellen können in Krebszellen entarten.

**Tabelle 2**

| | n²⁾ | "Sunborn-cells" pro 1 mm Epidermis (x ± s) | p³⁾ |
|---|---|---|---|
| Salbengrundlage + UV-Licht¹⁾ | 4 | 5,71 ± 1,23 | |
| Magnesiumcreme⁴⁾ + UV-Licht | 4 | 3,03 ± 1,07 | 0,05 |

| | | | |
|---|---|---|---|
| 1) 28 Behandlungstage, UV-Gesamtbestrahlungsdosis: 7,87 J/cm² | | | |
| 2) n = Anzahl der Patienten (der histologischen Untersuchungen nach 28 Behandlungstagen) | | | |
| 3) Statistische Signifikanz der Differenz (Mann-Whitney-Test) | | | |
| 4) Wogederm^{R} Magnesium-Creme | | | |

### Beispiel 3

Verminderte Ausprägung des UV-B-Licht-induzierten Erythems nach äußerlicher Behandlung der menschlichen Haut mit Magnesiumionen (Tabelle 3)

Zur Beweisführung wurde auf ein Hautareal von 2 cm x 2 cm bei freiwilligen Probanden 30 min vor einer UV-B-Testbestrahlung (0,83 mW/cm²) entweder allein die Salbengrundlage oder Wogederm^{R} Magnesium-Creme aufgetragen. Nach 24 h wurde das UV-B-induzierte Erythem bestimmt. Wie ersichtlich, resultierte als Folge der Wirkung der Magnesiumionen eine durchschnittliche Erythemabschwächung um den Faktor 3,73 (Erythemschutz-Faktor). Wurde die Magnesiumcreme 1 h vor der UV-B-Testbestrahlung aufgetragen, resultierte unter gleichen Bedingungen ein Erythemschutzfaktor von 4,48.

**Tabelle 3**

| Proband Nr. 2) | Minimale Erythemdosis (J/cm²⁾ | | Erythemschutz-Faktor (ESF) |
|---|---|---|---|
| | Salbengrundlage | Magnesiumcreme¹⁾ | |
| 1 | 0,015 | 0,060 | 4,0 |
| 2 | 0,015 | 0,050 | 3,3 |
| 3 | 0,015 | 0,040 | 2,7 |
| 4 | 0,010 | 0,100 | 10,0 |
| 5 | 0,005 | 0,005 | 1,0 |
| 6 | 0,005 | 0,015 | 3,0 |
| 7 | 0,005 | 0,015 | 3,0 |
| 8 | 0,001 | 0,020 | 20,0 |
| 9 | 0,001 | 0,001 | 1,0 |
| 10 | 0,005 | 0,015 | 3,0 |
| 11 | 0,015 | 0,015 | 1,0 |
| 12 | 0,005 | 0,010 | 2,0 |
| 13 | 0,080 | 0,080 | 1,0 |
| 14 | 0,015 | 0,020 | 1,3 |
| 15 | 0,015 | 0,030 | 2,0 |
| 16 | 0,005 | 0,015 | 3,0 |
| 17 | 0,080 | 0,120 | 1,5 |
| x ± s 0,017 ± 0,024 0,036 ± 0,035 3,73 ± 4,85 | | | |

| | | | |
|---|---|---|---|
| 1) Wogederm^{R} Magnesium-Creme | | | |
| 2) UV-Bestrahlung mit 0,83 mW/cm² und Ablesung nach 24 h | | | |
| 3) Applikation der Magnesiumcreme 30 min vor der UV-B-Lichttestung | | | |

### Beispiel 4

Folgende Komponenten wurden miteinander vermischt:

| | |
|---|---|
| dl-alpha-Tocopherol | 5,00 g |
| Magnesiumchlorid | 5,00 g |
| Miglyol | 15,00 g |
| Wasser | 5,00 g |
| Lanette Salbengrundlage | 70,00 g |
| | 100,00 g |

Diese Mischung ergab eine Creme, die sich gut auf der Haut verstreichen läßt und sehr rasch in die Haut eindringt. Sie gibt einen nicht mehr mit Wasser abwaschbaren nachhaltigen Schutz gegen UV-Licht mit hohem Lichtschutzfaktor, wobei die Wirkung der beiden Komponenten auf völlig verschiedenen Wirkmechanismen beruht. Entsprechend gute Ergebnisse sind erzielbar mit Rezepturen, in denen der Gehalt an dl-alpha-Tocopherol zwischen 3 und 10 g und der Gehalt an Magnesiumchlorid ebenfalls zwischen 3 und 10 g varriert wird, wobei der Wassergehalt etwa dem Gehalt an Magnesiumchlorid angepaßt werden kann.

## Patentansprüche

1. Verwendung von 0,5 bis 30 Gew.-% wasserlöslichen Magnesiumsalzen zur Herstellung von äußerlichen vorbeugenden Zubereitungen gegen UV-Licht-induzierte Schäden der Haut, insbesondere die vorzeitige Alterung und die Entstehung von Hautkrebs.

2. Verwendung gemäß Anspruch 1, dadurch gekennzeichnet, daß die Zubereitungen Magnesiumchlorid enthalten.

3. Verwendung gemäß einem der Ansprüche 1 oder 2, dadurch gekennzeichnet, daß die Zubereitungen zusätzlich 0,4 bis 25 Gew.-% Vitamin E oder Vitamin E Ester enthalten.

4. Äußerliche kosmetische vorbeugende Zubereitungen zur Abschwächung UV-Licht-induzierter Schäden der Haut, insbesondere die vorzeitige Alterung und die Entstehung von Hautkrebs, enthaltend
a) 0,5 bis 30 Gew.-% wasserlösliche Magnesiumsalze,
b) 0,4 bis 25 Gew.-% Vitamin E und/oder Vitamin E Ester.

## Claims

1. Use of 0.5 to 30% by weight of water-soluble magnesium salts for the preparation of topical formulations for preventing UV light induced damage to the skin, especially early ageing and the formation of cancer of the skin.

2. The use according to claim 1, characterized in that said formulations contain magnesium chloride.

3. The use according to any of claims 1 or 2, characterized in that said formulations additionally contain from 0.4 to 25% by weight of vitamin E or vitamin E ester.

4. Topical cosmetic preventive formulations for reducing UV light induced damage to the skin, especially early ageing and the formation of cancer of the skin, containing:
a) from 0.5 to 30% by weight of water-soluble magnesium salts;
b) from 0.4 to 25% by weight of vitamin E and/or vitamin E ester.

## Revendications

1. Utilisation de 0,5 à 30 % en poids de sels de magnésium solubles dans l'eau pour la fabrication de préparations destinées à un usage externe préventif contre les dommages cutanés induits par les rayons UV, en particulier le vieillissement prématuré et la survenance d'un cancer de la peau.

2. Utilisation selon la revendication 1, caractérisée en ce que les préparations contiennent du chlorure de magnésium.

3. Utilisation selon l'une des revendications 1 ou 2, caractérisée en ce que les préparations contiennent en plus 0,4 à 25 % en poids de vitamine E ou d'ester de vitamine E.

4. Préparations cosmétiques destinées à un usage externe préventif en vue de réduire les dommages cutanés induits par les rayons UV, en particulier le vieillissement prématuré et la survenance d'un cancer de la peau, et contenant
a) 0,5 à 30 % en poids de sels de magnésium solubles dans l'eau,
b) 0,4 à 25 % an poids de vitamine E et/ou d'ester de vitamine E.
